Europäisches Patentamt

European Patent Office

Office européen des brevets

(11) Publication number: **0 429 077 A2**

(12) **EUROPEAN PATENT APPLICATION**

(21) Application number: 90122268.7

(22) Date of filing: 22.11.90

(51) Int. Cl.⁵: **C12P 19/14**

(30) Priority: 24.11.89 JP 303351/89
24.11.89 JP 303352/89

(43) Date of publication of application:
29.05.91 Bulletin 91/22

(84) Designated Contracting States:
BE CH DE FR GB LI

(71) Applicant: MITSUI TOATSU CHEMICALS,
INCORPORATED
2-5, 3-chome, Kasumigaseki
Chiyoda-ku Tokyo(JP)

(72) Inventor: Tamatani, Hiroaki
18, Minami 12-chome, Higashi 4-jou
Sunagawa-shi, Hokkaido(JP)
Inventor: Okuno, Hiroyuki
5-6-305, Asahi-cho
Suita-shi, Osaka-fu(JP)
Inventor: Takahashi, Kazuo
3-2, Minami 10-chome, Higashi 3-jou
Sunagawa-shi, Hokkaido(JP)
Inventor: Yoshimi, Fuminobu
575-77, Aza Naie, Naie-cho
Sorachi-gun, Hokkaido(JP)
Inventor: Fukuoka, Akio
3-4-24, Ninami 12-chome, Higashi 4-jou
Sunagawa-shi, Hokkaido(JP)
Inventor: Sato, Kazuo
2-1, Minami 11-chome, Hinode 1-jou
Sunagawa-shi, Hokkaido(JP)

(74) Representative: Schüler, Horst, Dr.
Kaiserstrasse 69
W-6000 Frankfurt/Main 1(DE)

(54) Process for producing inulooligosaccharides.

(57) An inulooligosaccharide is produced by enzymatic decomposition of inulin or an inulin-containing plant with an inulase in which the enzymatic decomposition is carried out by using a reaction liquid containing 50 - 70 % by weight of water and/or the enzymatic decomposition is carried out at a temperature higher than a temperature range at which the enzyme is stable.

## PROCESS FOR PRODUCING INULOOLIGOSACCHARIDES

## BACKGROUND OF THE INVENTION

### Field of the Invention

The present invention relates to an improvement in a process for producing inulooligosaccharides by enzymatic decomposition of inulin.

### Description of Related Art

Inulooligosaccharides are oligosaccharides in which 2-5 molecules of fructose moieties are linked to each other by a $\beta$-2, 1-bond.

For example, these include inulotriose ($F_3$), inulotetraose ($F_4$), inulopentaose ($F_5$) and inulohexaose ($F_6$).

An inulooligosaccharide usually contains about 10 % by weight of fructooligosaccharides each having a glucose residue at the end.

It is said that the inulooligosaccharide can activate macrophages, multiply bifido bacteria and decrease fat in stock. Actually it is reported that when inulooligosaccharides are added to stock feed for raising, diarrhea in livestock can be inhibited and the feed efficiency is improved.

It is known that inulooligosaccharides can be usually obtained by hydrolyzing inulin or inulin-containing plant with acid or enzyme.

However, hydrolysis by acid has drawbacks that a large amount of fructose is formed in the decomposed product and the yield of inulooligosaccharide is lowered. Therefore, it is desired to produce inulooligosaccharides by enzymatic decomposition.

In conventional methods of enzymatic decomposition, as inulin-containing plants, there are used, for example, tubers of daliah and Jerusalem artichoke, chicory roots and the like, and as enzymes capable of decomposing inulin, there are known enzymes produced by fungi such as Aspergillus genus, Penicillium genus, and the like, yeasts such as Kluyveromyces genus, Candida genus and the like, and bacteria such as Bacillus genus and the like.

In order to produce inulooligosaccharides by decomposing the above-mentioned inulin-containing plants with such enzymes as above, the inulin-containing plants are chopped and then ground to be formed into slurry, and water is added or not added followed by enzymatic decomposition.

However, since such plants are used as the raw materials, there are various problems, that is, a large amount of the raw materials are concentrated at the harvest, the cost for transporting the raw materials is high, and saccharides decrease during storage. Therefore, the plants are chopped and then dried to decrease the water content to about 10 % by weight, and thereby the plant can be stored for a long time. Thus the above-mentioned problems can be solved by converting the plant to a dried matter.

Such a procedure is usually employed and desired for inulin raw materials.

In conventional enzymatic decomposition process, when the dried product is finely divided and water is added to the resulting finely divided dried product to form a slurry, the water content of the substrate is usually made high so as to effectively carry out the enzymatic reaction and the enzymatic decomposition is usually effected with a water content over 70 % by weight.

However, when the enzymatic decomposition is conducted at such a high water content, a large amount of water should be removed upon drying after the enzymatic decomposition. Therefore, investment of drying apparatus and running cost are raised, so that inulooligosaccharides can not be produced at a low cost disadvantageously.

According to the enzymatic decomposition process, optimum temperatures for most of such inulases originating from such microorganisms as above are 45 - 55° C, and it is reported that the inulases are usually rapidly deactivated at a temperature higher than 60° C.

Further, in the industrial process it is required to keep the reaction temperature at about 60° C or higher so as to avoid contamination with various other germs during the enzymatic decomposition, and heretofore a method has been proposed which comprises selecting a strain capable of producing a heat resistant enzyme not deactivated at 60° C (Japanese Patent Application Laid-open No. 9695/1983).

However, this method necessitates a very long time and labor to select such strain and has not yet obtained a sufficient result.

With respect to inulase, it is known that the enzyme stability increases when a substrate (for example, inulin and the like) is present upon the enzymatic reaction. This means that the lowering of the remaining activity of the enzyme is suppressed at a certain temperature with the lapse of time. but this does not indicate that an enzymatic reaction is effected at a temperature higher than a temperature range where the enzyme is stable. Japanese Patent Application Laid-open No. 9695/1983 discloses that an inulase produced by Aspergillus ficuum is used and an enzymatic decomposition is effected in the stable temperature region for enzyme (65° C or less), that is, 60° C.

J. Ferment. Technol., 66, 553 (1988) discloses that an inulase produced by Chrysosporium pannorum is used for an enzymatic decomposition reaction at 50°C within a stable temperature region for enzyme (50°C or less).

Japanese Patent Application Laid-open No. 136929/1977 discloses that an enzyme acts on an aqueous solution (85 % or more of water) extracted from Jerusalem artichoke with a warm water.

Further, Japanese Patent Application Laid-open No. 47481/1982 discloses that a surfactant is added to a water-insoluble substrate to effect an enzymatic decomposition.

## SUMMARY OF THE INVENTION

An object of the present invention is to enhance the efficiency of drying enzymatic decomposition products while keeping the enzymatic decomposition efficiency of inulin or an inulin-containing plant at a high level.

Another object of the present invention is to decrease the manufacturing cost of inulooligosaccharides.

A further object of the present invention is to improve the production efficiency of inulooligosaccharides.

Still another object of the present invention is to avoid contamination with various germs during enzymatic decomposition of inulin or an inulin-containing plant.

A still further object of the present invention is to enhance the content of inulooligosaccharides in the solid matter.

According to one aspect of the present invention, there is provided a process for producing an inulooligosaccharide comprising enzymatic decomposition of inulin or an inulin-containing plant with an inulase which comprises effecting the enzymatic decomposition by using a reaction liquid containing 50 - 70 % by weight of water.

According to another aspect of the present invention, there is provided a process for producing an inulooligosaccharide comprising enzymatic decomposition of inulin or an inulin-containing plant which comprises effecting the enzymatic decomposition at a temperature higher than a temperature range at which the enzyme is stable.

According to a further aspect of the present invention, there is provided a process for producing an inulooligosaccharide comprising enzymatic decomposition of inulin or an inulin-containing plant with an inulase which comprises effecting the enzymatic decomposition by using a reaction liquid containing 50 - 70 % by weight of water at a temperature higher than a temperature range at which the enzyme is stable.

## BRIEF DESCRIPTION OF THE DRAWING

FIG. 1 is a graph showing a relationship between a reaction temperature of an endo-type inulase produced by Penicillium purpurogenum var. rubri-sclerotium (FERM P-8705) * in the abscissa and a relative activity in the ordinate and an optimum reaction temperature is shown.

FIG. 2 is a graph showing temperatures at which the endo-type inulase is kept, in the abscissa and a remaining activity in the ordinate.

## DESCRIPTION OF THE PREFERRED EMBODIMENTS

According to the first aspect of the present invention, as inulin or an inulin-containing plant, there may be mentioned dahlia, tuber of Jerusalem artichoke, chicory roots and the like, and preferably the dried products therefrom are used.

Strains capable of producing enzymes used for the present invention may be any strains which can produce an inulase, and include strains of Aspergillus, Penicillium, Kluyveromyces, Candida, Bacillus, Saccharomyces, Fusarium, Talaromyces, Chaetomium, Pichia, and Chrysosporium genera.

For example, there may be mentioned Saccharomyces fragilis, Candida kefyr, Aspergillus niger, Aspergillus phoenicis, Aspergillus ficuum, Fusarium roseum, Talaromyces flavus, Chaetomium subspirale, Pichia polymorpha, Kluyveromyces marxianus, Bacillus circulans, Chrysosporium pannorum, and the like.

In particular, an endo-type inulase produced by Penicillium purpurogenum var. rubri-sclerotium is preferable, and an inulase produced by Aspergillus ficuum is also preferable.

A culture medium composition for cultivating strains may be composed of carbon source such as inulin, fructose, glucose, maltose and the like, nitrogen source such as yeast extract, corn steep liquor, inorganic salts and the like and minerals, and the cultivation is effected at 25 - 35°C for 2 - 5 days by aeration.

After completion of the cultivation, the filtrate is used as an enzyme solution. As a vessel for enzyme decomposition, a constant temperature vessel having an equipment capable of stirring a slurry

*) The domestic deposit of Penicillium purpurogenum var. rubri-sclerotium (FERM P-8705, deposited on March 20, 1986) was converted to a deposit under Budapest Treaty on November 13, 1990. The Acession (Acceptance) Number given by Fermentation Research Institute , Agency of Industrial Science and Technology, 1-3, Higashi 1-chome, Tsukuba-shi, Ibaraki-ken 305, Japan, is FERM BP-3162.

in a vessel is the most suitable, and a concrete mixer or ribbon mixer provided with a thermostat can be also used.

An enzyme-containing liquid is added to a mixture of a finely divided dried inulin-containing plant and a warm water at 40 - 60°C and placed in a decomposition vessel followed by uniform mixing.

The water content here is 50 - 70 % by weight. When the water content is less than 50 % by weight, undecomposed inulin remains in a large amount, and the yield of inulooligosaccharide, the end product, is lowered. On the contrary, when the water content exceeds 70 % by weight, the enzymatic decomposition reaction proceeds sufficiently, but the drying cost of the decomposed product is disadvantageously high.

Thus, after the enzymatic decomposition at 40 -70°C for 12 - 36 hours in a decomposition vessel, the product is dried by using an ordinary drum-type drier or a blowing drier up to an water content of 5 % by weight or less and as a result, a product can be obtained which contains 40 % by weight (inulin decomposition rate, 57 %) or more of inulooligosaccharide based on the solid product.

According to the second aspect of the present invention, an inulooligosaccharide is produced by enzymatic decomposition of inulin or an inulin-containing plant at a temperature higher than a temperature range at which the enzyme is stable.

In particular, the enzymatic decomposition reaction is carried out at a temperature ranging from 55 to 70°C which is higher than a temperature range where the enzyme is stable.

The enzymes and the culture medium for cultivating a strain capable of producing an inulase used in the first aspect may be also used here.

The strain is usually subjected to an aerated cultivation at 25 - 35°C for 2 - 5 days and after completion of the cultivation, the microorganism is removed by filtration to give a crude enzyme solution.

The resulting inulase can be recovered from the crude enzyme solution and purified according to a conventional method, for example, the purification can be carried out by using an ion exchange resin or gel filtration.

The optimum reaction temperature of the inulase thus purified varies depending on a strain used, but is usually 45 - 60°C and the stable temperature of enzyme is usually lower than 60°C.

For purposes of producing inulooligosaccharides, an endo-type inulase is preferable. When an enzyme solution having an endo/exo ratio of I/S >10 where I is an inulin decomposition activity and S a sucurose decomposition activity is used, the production of fructose is suppressed and an inulooligosaccharide can be effectively produced.

Therefore, if necessary, a part of the crude enzyme solution is purified for removing the exo-type inulase to obtain an enzyme solution of high I/S, and then the resulting enzyme solution is used for the production of inulooligosaccharide.

Methods for measuring the optimum reaction temperature and stable temperature of inulase are as shown below.

The optimum reaction temperature is determined by using 1.0 ml of a 0.5 weight % solution of inulin [M/10 acetic acid buffer solution (pH 5.0)] and 1.0 ml of a diluted enzyme solution, carrying out the reaction at each temperature for 30 min. and effecting a quantitative colorimetric analysis of the resulting reducing sugars by the 3,5-dinitrosalicylic acid method to obtain the relative activity.

The stable temperature of enzyme is determined by keeping 1.0 ml of a diluted enzyme solution at each temperature for 10 min., adding 1.0 ml of a 0.5 weight % solution of inulin [M/10 acetic acid buffer solution (pH 5.0)] thereto, carrying out the reaction at 50°C for 30 min. and conducting a quantitative colorimetry of the resulting reducing sugar to determine the remaining activity.

A process for producing an inulooligosaccharide from an inulin-containing plant such as tuber of Jerusalem artichoke, chicory roots and the like is exemplified below.

Firstly an inulin-containing plant is chopped and ground to form a slurry, or a dried inulin-containing plant is finely divided and water is added thereto to form a slurry. To the slurry is added uniformly the above-mentioned enzyme solution to form a mixture containing 50 - 85 wt. % of water and an enzymatic decomposition is carried out for 12 - 36 hours.

The decomposing temperature is set at a temperature in the range of 55 - 70°C simultaneously with a temperature higher than a stable temperature of inulase by 10°C or less.

Thus, the production of inulooligosaccharide is increased and contamination with various germs can be lessened.

By drying enzymatic decomposition products up to 5 % by weight of water or less by an ordinary drum-type drier or a blower drier, there is obtained a product containing 40 % by weight (inulin decomposition rate, 57 %) or more of inulooligosaccharide based on the solid matter.

In such a way as above, an enzymatic decomposition at a temperature higher than a stable temperature of inulase results in a high production rate of inulooligosaccharide as compared with an enzymatic decomposition at a temperature lower than the stable temperature of inulase. As a result, the amount of enzyme to be used can be reduced so that it serves to decrease the production cost of inulooligosaccharide.

According to the third aspect of the invention, the above-mentioned two conditions, i.e. 50 - 70 % by weight of the water content in the reaction liquid and enzymatic decomposition at a temperature higher than the stable temperature of the enzyme, can be employed in combination for producing an inulooligosaccharide by enzymatic decomposition of inulin or an inulin-containing plant with an inulase.

As mentioned above, according to the present invention, a process for producing an inulooligosaccharide by enzymatic decomposition of inulin or an inulin-containing plant is improved and an inulooligosaccharide can be efficiently produced.

In particular, according to the first aspect, the drying efficiency of the enzymatic decomposition product can be enhanced while keeping the enzymatic decomposition efficiency at a high level. Further, when the enzymatic decomposition is effected at a low water content, an inulooligosaccharide can be efficiently produced and thereby the drying cost after enzymatic decomposition can be decreased to a great extent. An inulooligosaccharide can be industrially produced at a low cost.

According to the second aspect, enzymatic decomposition of inulin or an inulin-containing plant is carried out at a temperature higher than the stable temperature of an inulase so that the inulooligosaccharide content based on the solid matter can be enhanced and thereby the production rate can be increased. In addition, such a high temperature enzymatic decomposition serves to lessen contamination with various germs.

Further, according to the third aspect, the advantageous effects of the first and the second aspects can be obtained in combination.

The present invention is explained more in detail by referring to the following examples, but the sxamples should not be construed as limitation to the present invention.

EXAMPLE 1
(1) Preparation of Inulase

As a strain, there was used Penicillum purpurogenum var. rubri-sclerotium (FERM P-8705). As a culture medium, inulin 0.5 wt. %, maltose 0.5 wt. %, $NH_4H_2PO_4$ 0.3 wt. %, $KH_2PO_4$ 0.1 wt. %, Tween 80 0.02 wt. %, and $MgSO_4 \cdot 7H_2O$ 0.05 wt. % were dissolved in tap water and adjusted to pH 7.0, and 100 ml of the resulting culture medium was placed in a Sakaguchi flask, sterilized, then inoculated with one platinum loop of the strain and pre-cultivated at 28°C for 2 days.

The same culture medium (3.5 liters) as above placed in a 5-liter cultivating vessel was inodulated with the pre-cultivated liquid as above, and cultivated at 28°C for 4 days. The resulting filtrate after cultivation was used as an enzyme solution.

(2) Preparation of Inulooligosaccharide

As an inulin-containing plant, a dried powder of chicory roots (hereinafter called "chicory powder") was used.

The amount of the enzyme solution used was 2 units per 1 g of chicory powder and the enzyme activity was measured by the following method.

To 0.5 ml of a solution prepared by dissolving 1.5 wt. % of inulin in a M/10 acetic acid buffer solution (pH 5.0) was added 0.5 ml of the enzyme solution diluted with the buffer solution followed by causing the reaction at 50°C for 30 min. and the resulting reducing sugar was subjected to a quantitative colorimetry according to the 3,5-dinitrosalicylic acid method.

An amount of an enzyme capable of producing 1μmol. of a reducing sugar per min. was designated as one unit.

The enzymatic decomposition was carried out by using a 200 l. concrete mixer (manufactured by MEIHO) and throwing 70 kg of chicory powder little by little into the mixer while rotating the agitating vanes, simultaneously with adding uniformly a warm water mixed with the enzyme solution at 60°C thereto by using a spray so as to adjust the water content in the reaction material to 60 % by weight.

And without interruption, an enzymatic decomposition was carried out at 60°C for 20 hours and the resulting decomposition product was dried at 80 - 100°C up to a water content of 3 % by weight or less, and then the product was pulverized to 20 mesh or smaller size to obtain 55 kg of inulooligosaccharide-containing powder. The content of inulooigosaccharide in the solid matter of the powder was 54.9 % (inulin decomposition rate, 78 %).

EXAMPLE 2
(1) Preparation of Inulase

The procedure of Example 1(1) was repeated to prepare a crude enzyme solution.

The crude enzyme solution (1 liter) was subject to salting out, ·passed through a DEAE-Sepharose CL-6B (tradename, supplied by Pharmacia) column, eluted with NaCl to collect active fractions of an endo-type inulase, and then the fractions were subjected to a gel filtration by using Sephacryl S-200 (tradename, supplied by Pharmacia) for purifying until substantially a single band can be obtained from a standpoint of electrophoresis.

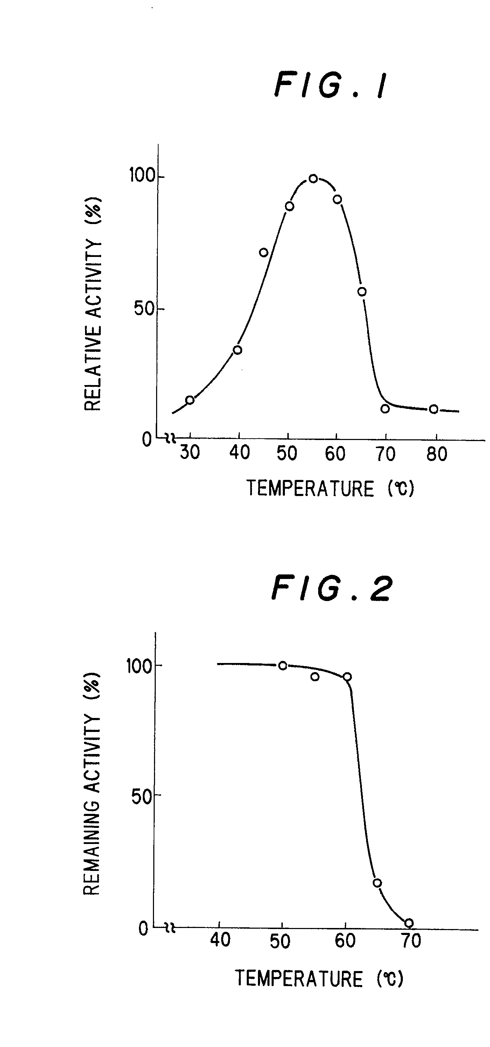

The endo-type inulase thus purified has an optimum reaction temperature at 50 - 60°C and a stable temperature lower than 60°C (see Fig. 1 and Fig. 2).

(2) Preparation of Inulooligosaccharide

kg of chicory roots was chopped and ground to form a slurry (water content, 75 % by weight). To the resulting slurry was added 10000 units of the above-mentioned crude enzyme solution and mixed followed by enzymatic decomposition at 65°C for 18 hours.

The product was dried and groud to obtain a powder containing 68 % by weight (inulin decomposition rate, 90 %) of inulooligosaccharide based on the solid matter.

## COMPARATIVE EXAMPLE 1

The prodedure of Example 2 was repeated except that the enzymatic decomposition temperature was 58°C, and the resulting powder contained 62 % by weight (inulin decomposition rate, 82 %) of inulooligosaccharide based on the solid matter.

## COMPARATIVE EXAMPLE 2

The procedure of Example 2 was repeated except that the crude enzyme liquid corresponding to 10000 units was directly used without adding the chicory slurry and kept at 65°C for 10 min., and the resulting enzyme solution was used for enzymatic decomposition. The content of inulooligosaccharide in the resulting powder was 38 % by weight based on the solid matter.

## EXAMPLE 3

(1) Preparation of Inulase

As a strain, Aspergillus ficuum, AHU 7015 (deposited at Department of Agricultural Chemistry, Faculty of Agriculture, Hokkaido University, Nishi 9-chome, Kita Kyujo, Kita-ku, Sapporo-shi, Hokkaido, Japan ; available on request) was used and the cultivation was carried out following the procedure of Example 1.

After completion of the cultivation, the product was filtered to remove the strain, and ammonium sulfate was added to the resulting filtrate. The resulting precipitate from a solution of 60 - 85 % saturation of ammonium sulfate was collected and subjected to dialysis with a M/100 acetic acid buffer solution (pH 5.0) to obtain a crude enzyme solution.

The stable temperature of the endo-type inulase was 50°C or lower.

(2) Preparation of Inulooligosaccharide

The procedure of Example 2 was repeated by using chicory roots for enzymatic decomposition. The inulooligosaccharide content produced by decomposition at 55°C was about 1.2 times that produced by decomposition at 50°C.

## EXAMPLE 4

The procedure of Example 1 was repeated to prepare an inulase and the enzymatic reaction was carried out using a 1 m³ ribbon mixer. That is, 200 l. of tap water and 30 l. of the crude enzyme solution was placed in the ribbon mixer and 150 kg of a chicory powder was uniformly mixed therewith followed by an enzymatic decomposition at 65°C for 20 hours.

The water content upon the enzymatic decomposition was 65 % by weight, and after the decomposition, the product was dried and pulverized. The resulting powder contained inulooligosaccharide in an amount of 61 % by weight based on the solid matter.

## Claims

1. A process for producing an inulooligosaccharide comprising enzymatic decomposition of inulin or an inulin-containing plant with an inulase which comprises effecting the enzymatic decomposition by using a reaction liquid containing 50 - 70 % by weight of water.

2. The process according to claim 1 in which the enzyme is an inulase produced by Penicillium purpurogenum var. rubri-sclerotium or Aspergillus ficuum.

3. A process for producing an inulooligosaccharide comprising enzymatic decomposition of inulin or an inulin-containing plant which comprises effecting the enzymatic decomposition at a temperature higher than a temperature range at which the enzyme is stable.

4. The process according to claim 3 in which the enzymatic decomposition reaction is carried out at 55 - 70°C.

5. The process according to claim 3 in which the enzyme is an inulase produced by Penicillium purpurogenum var. rubri-sclerotium or Aspergillus ficuum.

6. A process for producing an inulooligosaccharide comprising enzymatic decomposition of inulin or an inulin-containing plant with an inulase which comprises effecting the enzymatic decomposition by using a reaction liquid containing 50 - 70 % by weight of water at a temperature higher than a temperature range at which the enzyme is stable.

7. The process according to claim 6 in which the enzymatic decomposition reaction is carried out at 55 - 70°C.

8. The process according to claim 6 in which the enzyme is an inulase produced by *Penicillium purpurogenum* var. *rubri-sclerotium* or *Aspergillus ficuum*.

# FIG. 1

# FIG. 2